# FASCICULE DE BREVET EUROPEEN

(11) **EP 0 942 001 B1**
(45) Date de publication et mention de la délivrance du brevet: **23.05.2001**
(21) Numéro de dépôt: 99400315.0
(22) Date de dépôt: 10.02.1999
(51) Int. Cl.: C07J 41/00, A61K 31/575

(54) **Composés lipidiques dérivés des bases sphingoides, leur procédé de préparation et leurs utilisations en cosmétique et en dermopharmacie**
Sphingoid Lipidverbindungen, ein Verfahren zur ihrer Herstellung und kosmetische und dermopharmazeutische Verwendungen davon
Sphingoid Lipid Compounds, a Process for Their Production and Cosmetic and Dermopharmaceutical uses Thereof

(30) Priorité: 10.03.1998 FR 9802915
(43) Date de publication de la demande: 15.09.1999
(73) Titulaire: L'OREAL, 75008 Paris (FR)
(72) Inventeur: Semeria, Didier, 71181 Courtry (FR); Philippe, Michel, 91320 Wissous (FR)
(74) Mandataire: Dodin, Catherine

(56) Documents cités:
- EP-A- 0 420 722
- EP-A- 0 890 577

## Description

La présente invention a pour objet de nouveaux composés lipidiques dérivés de bases sphingoïdes, leur procédé de préparation ainsi que leur utilisation, notamment pour les traitements et les soins de la peau, des cheveux, des ongles et des cils en cosmétique ou en dermatologie.

L'exposition de la peau au froid, au soleil, aux atmosphères à faible humidité relative, les traitements répétés avec des compositions de lavage ou encore le contact avec des solvants organiques, sont des facteurs qui entraînent, à des degrés divers, un dessèchement apparent. La peau apparaît plus sèche, moins souple et le relief cutané plus prononcé. Par ailleurs, les cheveux, qui sont soumis trop fréquemment à certains traitements capillaires, perdent leur aspect brillant et peuvent devenir rêches et cassants.

La demanderesse a donc recherché des composés qui permettent de prévenir ou de corriger ces phénomènes se traduisant par un dessèchement apparent et qui redonnent à la peau sa souplesse et aux cheveux leur brillance et leur douceur.

Pour résoudre ce problème, on a déjà proposé d'utiliser des composés lipidiques, notamment dans le brevet français 2 652 002. Ces composés, utilisés dans des compositions cosmétiques ou dermatologiques pour les traitements et les soins de la peau et des cheveux ont un effet hydratant permettant de prévenir ou de corriger certains effets du dessèchement apparent de la peau ou des cheveux. Toutefois, il serait souhaitable de mettre au point des composés qui, utilisés dans des compositions cosmétiques ou dermatologiques, aient un effet hydratant ou traitant supérieur à celui des composés de ce brevet.

Le document EP-A-0 890 577 opposable selon l'article 54(3) CBE, décrit une composition comprenant une dispersion aqueuse de vésicules lipidiques à base de carbamate à chaîne cholestéryle et son utilisation topique.

La présente invention a donc pour objet de nouveaux composés lipidiques dérivés de bases sphingoïdes répondant à la formule : 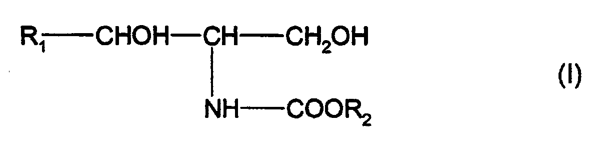 dans laquelle
* R₁ désigne un radical hydrocarboné, en particulier un radical alkyle, linéaire ou ramifié, saturé ou insaturé pouvant être substitué par un ou plusieurs hydroxyles éventuellement estérifiés, en C1-C31, et de préférence en C9-C25;
* OR₂ désigne un radical cholestéryle; lesdits composés étant sous forme d'énantiomère pur ou sous forme de mélange d'isomères, à l'exception des composés pour lesquels R1 désigne un radical alkyle linéaire ou ramifié, saturé ou insaturé, éventuellement hydroxylé ayant de 1 à 3 atomes de carbone.

Parmi les composés préférentiels de formule (I), on peut citer :
- le N-(cholestéryloxycarbonyl)-2-amino-octadécane-1,3-diol ;
- le N-(cholestéryloxycarbonyl)-2-amino-octadécane-1,3,4-triol ;
lesdits composés étant sous forme pure ou sous forme de mélanges d'isomères.

Les composés lipidiques de formule (I) ci-dessus sont, par leur structure, très proches des céramides qui sont les éléments constitutifs prépondérants des lipides intercornéocytaires du stratum cornéum. On admet généralement qu'ils participent au maintien de l'intégrité de la barrière cutanée. On en trouve également à moindre degré, au niveau du cheveu.
Les composés de formule (I) selon l'invention présentent donc un intérêt tout particulier pour les traitements et les soins de la peau, des cheveux, des ongles et des cils en cosmétique ou en dermatologie dont les effets hydratants sont substantiellement plus importants que ceux apportés par les composés lipidiques dérivés de bases sphingoïdes de l'art antérieur notamment ceux du brevet français 2 652 002

Les composés lipidiques de formule (I) présentent une bonne innocuité vis à vis de la peau, des cheveux, des ongles, des cils, des sourcils et des muqueuses. Ils ont de bonnes propriétés émollientes et adoucissantes. Ils sont facilement solubilisés dans les phases grasses des préparations cosmétiques et dermopharmaceutiques.

De plus, les cheveux traités par ces composés de formule (I) présentent un aspect brillant, un toucher plus doux et une moins grande sensibilité à l'eau, due à l'apport de matière lipidique uniformément répartie sur les écailles du cheveu. Les propriétés mécaniques et de nervosité sont également améliorées.

Les composés selon l'invention peuvent former avec d'autres lipides des vésicules.

Les composés lipidiques selon l'invention de formule (I) ci-dessus peuvent être obtenus par la condensation, sur la fonction amine d'une base sphingoïde de formule (Il) suivante : 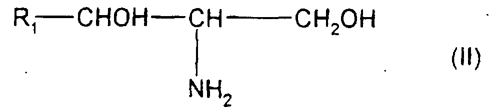 dans laquelle R₁ a la même signification indiquée dans la formule (I)
- soit d'un chloroformiate de cholestéryle de formule (III) suivante : 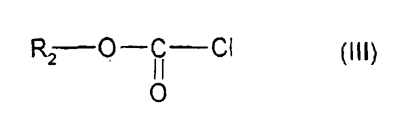 dans laquelle OR₂ a la même signification indiquée dans la formule (I) dans un solvant organique approprié en présence d'une base ;
- soit de l'imidazolide de cholestéryle de formule (IV) suivante : 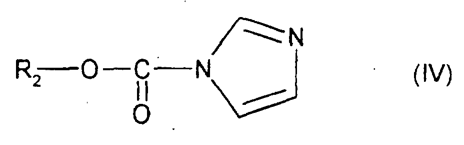 dans laquelle OR₂ a la même signification indiquée dans la formule (I).

Le procédé de préparation des composés de formule (I) peut être représenté par le schéma suivant : 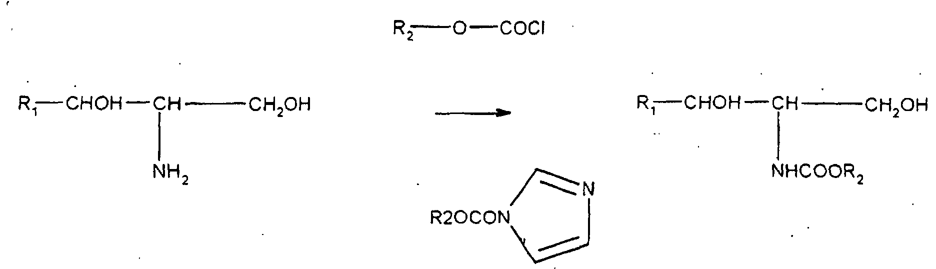 R₁ et OR₂ ayant les significations indiquées ci-dessus.

Les composés de formule (I) peuvent être obtenus soit par réaction des composés de formule (II) avec le chloroformiate (III) dans des solvants tels que le tertiobutylméthyléther, le diméthylformamide ou le tétrahydrofurane (THF) en présence d'une base comme par exemple la triéthylamine, la pyridine, le bicarbonate de sodium, etc. ; soit par réaction des composés de formule (Il) avec l'imidazolide (IV) isolé ou préparé in situ par exemple par réaction d'un alcool gras avec le carbonyldiimidazole.

Le chloroformiate de cholestéryle est un produit commercial. L'imidazolide de cholestéryle est synthétisé suivant les méthodes décrites par H. A. STAAB dans Angew. Chem. International Edit. vol. 1 (1962), n°7 p. 351.

Pour la préparation du composé de formule (I), on peut également utiliser le chlorhydrate du composé de formule (II).

Les composés de formule (Il) sont des composés connus. Leur synthèse a été décrite en particulier par D. SHAPIRO dans «Chemistry of sphingolipids», HERMANN, Paris (1969).

Les procédés de synthèse décrits ci-dessus conduisent à des mélanges racémiqùes. Il est possible d'obtenir des composés sous forme d'énantiomères purs en faisant une résolution du racémique décrite par SHAPIRO dans l'article précité à la page 99. On peut également synthétiser directement des énantiomères purs selon les procédés décrits par R. SCHMIDT dans «Tetrahedron Letters», vol. 27, no 4, pages 481-484 (1986) ; par B. BERNET dans la même revue vol. 24, no 49, pages 5491-5494 (1983) ; ou encore par MAKOTO KISO dans «Carbohydrate Research», 158 (1986), pages 101-111.

Les composés lipidiques selon l'invention de formule (I) peuvent recevoir des applications diverses, notamment dans des compositions cosmétiques et dermatologiques. Ces composés possèdent en plus la propriété de former des vésicules en association avec d'autres lipides, lorsqu'ils sont dispersés dans l'eau.

La présente invention a donc pour objet l'utilisation des composés de formule (I) dans des émulsions, des dispersions ou dans des lotions. Elle a également pour objet l'utilisation de ces composés, associés à d'autres lipides, pour la formation de sphérules lipidiques.

La présente invention a également pour objet des compositions à usage cosmétique ou dermatologique contenant dans un milieu cosmétiquement acceptable au moins l'un des composés de formule (I) tels que définis ci-dessus.

Un autre objet de l'invention est un procédé de traitement cosmétique de la peau, des cheveux, des ongles, des cils ou des sourcils consistant à appliquer sur ces derniers une quantité suffisante d'une telle composition contenant les composés de formule (I).

Les compositions selon l'invention peuvent se présenter sous forme d'émulsions (lait ou crème), de lotions hydroalcooliques, huileuses ou oléoalcooliques, de gels, de dispersions ou de bâtonnets solides, de sprays ou de mousse aérosol.

Selon l'invention, les composés de formule (I) représentent de 0,005% à 20%, de préférence de 0,01 à 10% du poids total de la composition.

Les compositions sont par exemple des lotions, des laits ou des crèmes émollients, des laits ou des crèmes pour les soins de la peau ou des cheveux, des crèmes, des lotions ou des laits démaquillants, des bases de fond de teint, des lotions, des laits ou des crèmes antisolaires, des lotions, des laits ou des crèmes de bronzage artificiel, des crèmes ou des mousses de rasage, des lotions après rasage, des shampooings, des rouges à lèvres, des mascaras ou des vernis à ongles.
Ces compositions peuvent également se présenter sous la forme de bâtons pour les lèvres destinés soit à les colorer, soit à éviter les gerçures, ou de produits de maquillage pour les yeux ou des fards et fonds de teint pour le visage.

Lorsque les compositions selon l'invention se présentent sous la forme d'émulsions de type eau-dans-huile ou huile-dans-eau, la phase grasse est essentiellement constituée d'un mélange de composés de formule (I) avec au moins une huile, et éventuellement un autre corps gras.

La phase grasse des émulsions peut constituer de 5 à 60% du poids total de l'émulsion.
La phase aqueuse desdites émulsions constitue de préférence de 30 à 85% du poids total de l'émulsion.
La proportion de l'agent émulsionnant peut être comprise entre 1 et 20%, et de préférence entre 2 et 12% du poids total de l'émulsion.

Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau; les lotions huileuses peuvent en outre constituer des huiles moussantes contenant un tensioactif oléosoluble, des huiles pour le bain, etc.

Parmi les principaux adjuvants pouvant être présents dans les compositions selon l'invention, on peut citer les corps gras tels que les huiles ou les cires minérales, animales ou végétales, les acides gras, les esters d'acide gras tels que les triglycérides d'acides gras ayant de 6 à 18 atomes de carbone, les alcools gras; les émulsionnants comme les alcools gras oxyéthylénés ou les alcoyléthers de polyglycérol; les solvants tels que les monoalcools ou polyalcools inférieurs contenant de 1 à 6 atomes de carbone ou encore l'eau.
Les mono- ou polyalcools plus particulièrement préférés sont choisis parmi l'éthanol, l'isopropanol, le propylèneglycol, le glycérol et le sorbitol.

A titre de corps gras, parmi les huiles minérales, on peut citer l'huile de vaseline; parmi les huiles animales, les huiles de baleine, de requin, de phoque, de menhaden, de foie de flétan, de morue, de thon, de tortue, de pied de boeuf, de pied de cheval, de pied de mouton, de vison, de loutre, de marmotte, etc.; parmi les huiles végétales, les huiles d'amande, de germes de blé, d'olive, de germe de maïs, de jojoba, de sésame, de tournesol, de palme, de noix, de karité, de shoréa, de macadamia, de pépins de cassis et similaires.
Parmi les esters d'acides gras, on peut utiliser des esters d'acides en C12 à C22 saturés ou insaturés et d'alcools inférieurs comme l'isopropanol ou le glycérol ou d'alcools gras en C8 à C22, linéaires ou ramifiés, saturés ou insaturés ou encore d'alcanediols-1,2 en C10 à C22.
On peut également citer comme corps gras, la vaseline, la paraffine, la lanoline, la lanoline hydrogénée, le suif, la lanoline acétylée, les huiles de silicone.
Parmi les cires, on peut citer la cire de Sipol, la cire de lanoline, la cire d'abeille, la cire de Candelilla, la cire microcristalline, la cire de Carnauba, le spermacéti, le beurre de cacao, le beurre de karité, les cires de silicone, les huiles hydrogénées concrètes à 25°C, les sucroglycérides, les oléates, myristates, linoléates et stéarates de calcium, magnésium et aluminium.
Parmi les alcools gras, on peut citer les alcools laurique, cétylique, myristique, stéarique, palmitique, oléique et les alcools de GUERBET comme le 2-octyldodécanol, le 2-décyltétradécanol ou le 2-hexyldécanol.

A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de polyglycérol, les alcools en C12 à C18 comportant de 2 à 10 moles de glycérol.
Il peut être aussi utile d'utiliser des épaississants tels que les dérivés de cellulose, les dérivés d'acide polyacrylique, les gommes de guar ou de caroube ou la gomme de xanthane.

La composition selon l'invention peut également contenir des adjuvants habituellement utilisés en cosmétique ou en dermatologie et notamment des produits hydratants, des adoucissants, des produits pour le traitement d'affections cutanées, des filtres solaires, des germicides, des colorants, des conservateurs, des parfums et des propulseurs.

Lorsque les compositions selon l'invention sont des dispersions, il peut s'agir de dispersions de composés de formule (I) dans l'eau en présence de tensioactif ou encore de dispersions aqueuses de sphérules lipidiques, constituées de couches moléculaires organisées enfermant une phase aqueuse encapsulée, ces couches étant constituées d'au moins un composé de formule (I) associé à au moins un autre composé lipidique.

On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les brevets français no 2315991, 1477048 et 2091516 ou dans les demandes de brevet international WO 83/01571 et WO 92/08685.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou non ionique. A titre de groupements non ioniques, on peut citer des groupements dérivés de polyéthylèneglycol. On peut aussi utiliser avantageusement comme lipides formant la phase lamellaire, des éthers de polyglycérol tels que ceux décrits dans les brevets français no 1477048, 2091516, 2465780 et 2482128.

A titre de groupement ionique, on peut avantageusement utiliser un groupement dérivé d'un composé amphotère, anionique ou cationique.

D'autres lipides décrits dans la demande de brevet international WO 83/01571 comme pouvant être utilisés pour la formation de vésicules sont les glycolipides comme le lactosylcéramide, le galactocérébroside, les gangliosides et le trihexosylcéramide, ainsi que les phospholipides tels que le phosphatidylglycérol et le phosphatidylinositol.

La présente invention a donc pour objet une dispersion de sphérules lipidiques constituées de couches moléculaires organisées de composés de formule (I) et de lipide défini ci-dessus renfermant une phase aqueuse à encapsuler.
La phase continue de la dispersion qui entoure les sphérules est une phase aqueuse.
Les sphérules en dispersion ont généralement un diamètre compris entre 0,05 microns et 5 microns.
La phase aqueuse encapsulée dans les sphérules peut être de l'eau ou une solution aqueuse de substance active et est dans ce cas de préférence isoosmotique par rapport à la phase continue de la dispersion.

Les sphérules peuvent être obtenues en particulier suivant le procédé décrit dans le brevet français 2315991 de la Demanderesse, selon lequel on prépare une dispersion de sphérules constituées de couches moléculaires organisées renfermant une phase aqueuse à encapsuler, en mettant en contact d'une part des mélanges d'isomères de composés de formule (I) associées à un ou plusieurs lipide(s) défini(s) ci-dessus et d'autre part la phase aqueuse à encapsuler dans les sphérules en agitant pour assurer le mélange et obtenir une phase lamellaire, en ajoutant ensuite un liquide de dispersion en quantité supérieure à la quantité de phase lamellaire obtenue et en secouant énergiquement pendant une durée allant de 15 minutes à 3 heures environ.
Un autre procédé de préparation peut consister à utiliser le procédé dénommé REV (reverse-phase évaporation vesicle) ou évaporation en phase inverse décrit. dans Proc. Natl. Acad. Sci. USA., Vol. 75, no 9, pages 4194-4198 (1978), par SZOKA et PAPAHADJOPOULOS.
On peut également mettre en oeuvre le procédé qui comprend la succession d'étapes consistant à dissoudre au moins un lipide dans au moins un solvant organique non miscible à l'eau ; ajouter la phase organique ainsi obtenue à une phase aqueuse ; former une dispersion des deux phases sous forte agitation, la taille des vésicules pouvant être réglée en faisant varier la vitesse d'agitation au cours de ce mélange de phase ; conduire l'évaporation du (ou des) solvant(s) sous forte agitation ; et, le cas échéant, concentrer la dispersion.

Les substances actives peuvent être des substances ayant un intérêt pharmaceutique, alimentaire ou des substances ayant une activité cosmétique. Lorsqu'elles sont hydrosolubles, elles sont dans la phase aqueuse encapsulée à l'intérieur des vésicules.
Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone carboxylique et ses sels; des agents de brunissage artificiel tels que la dihydroxyacétone, l'érythrulose, le glycéraldéhyde, les y-dialdéhydes tels que l'aldéhyde tartrique, ces composés étant éventuellement associés à des colorants; des filtres solaires hydrosolubles ; des antiperspirants, des déodorants, des astringents, des produits rafraîchissants, toniques, cicatrisants, kératolytiques, dépilatoires, des eaux parfumées ; des extraits de tissus végétaux, tels que les polysaccharides ; des colorants hydrosolubles ; des agents antipelliculaires; des agents antiséborrhéiques; des oxydants tels que des agents de décoloration comme l'eau oxygénée; des réducteurs tels que l'acide thioglycolique et ses sels.
On peut citer également les vitamines, les hormones, les enzymes telles que la superoxyde dismutase, les vaccins, les anti-inflammatoires tels que l'hydrocortisone, les antibiotiques, les bactéricides, les agents cytotoxiques ou anti-tumoraux.

Lorsque les substances actives sont liposolubles, elles se trouvent incorporées dans les feuillets des vésicules. Elle peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les rétinoïdes, les antioxydants, les acides gras essentiels, l'acide glycyrrhétinique, les kératolytiques et les caroténoïdes.

Les dispersions de sphérules lipidiques présentent l'intérêt de véhiculer des substances actives qui se trouvent ainsi masquées et protégées vis-à-vis des différents agents d'altération : oxydants et plus généralement composés réactifs vis-à-vis des substances actives encapsulées. La pénétration et la fixation des substances actives peuvent être modulées par la variation de la taille des sphérules et de leur charge électrique. L'action de ces substances actives peut également être ainsi différée (effet retard).

L'invention a enfin pour objet l'utilisation en cosmétique d'une dispersion aqueuse de sphérules constituée de couches moléculaires organisées de composés de formule (I) associés à d'autres lipides renfermant une phase aqueuse à encapsuler, en particulier pour le traitement de la peau.

L'invention a également pour objet l'utilisation d'une telle dispersion de sphérules lipidiques en dermatologie ou dans l'industrie alimentaire.

Dans ce qui suit ou ce qui précède, les pourcentages sont donnés en poids, sauf mention contraire.
Dans ces exemples, M.A. signifie matière active.

### Exemple 1 : Préparation du N-(cholesteryloxycarbonyl)-2-amino-octadécane-1,3-diol

70 g (0,23 mole) de 2-amino-octadécane-1,3-diol sont solubilisés à 35°C dans 1 litre de tertiobutylméthyléther. Après addition en 1 heure de 109 g (0,24 mole) de chloroformiate de cholestéryle et de 33,8 ml de triéthanolamine, le milieu réactionnel est agité pendant 4 heures à 35°C.
Après extraction de la phase organique, celle-ci est séchée, évaporée à sec. Le produit brut est cristallisé dans de l'acétone pour conduire à 114 g du produit attendu (rendement de 69%).

Le spectre RMN ¹³C et l'analyse élémentaire du produit obtenu sont conformes au produit attendu de point de fusion de 93-101°C.

| Analyse élémentaire : | | | | |
|---|---|---|---|---|
| | **C** | **H** | **N** | **O** |
| **Théorique** | 77,36 | 11,71 | 1,96 | 8,96 |
| **Trouvé** | 77,24 | 11,64 | 1,82 | 9,21 |

### Exemple 2 : Préparation du N-(cholesteryloxycarbonyl)-2-amino-octadécane-1,3,4-triol (4R. 3S,2S)

63 g (0,19 mole) de 2-amino-octadécane-1,3,4-triol (4R, 3S,2S), sont solubilisés partiellement à 35°C dans 800 ml de tétrahydrofurane. Après addition en 2 heures de 89,8 g (0,2 mole) de chloroformiate de cholestéryle et de 30 ml de triéthylamine, le milieu réactionnel est agité pendant 2 heures à 35°C.
Après extraction de la phase organique, celle-ci est séchée, évaporée à sec. Le produit brut est cristallisé dans de l'acétate d'éthyle pour conduire à 122 g du produit attendu (rendement de 83%).

Le spectre RMN ¹³C et l'analyse élémentaire du produit obtenu sont conformes au produit attendu de point de fusion de 135-148°C.

| Analyse élémentaire : | | | | |
|---|---|---|---|---|
| | **C** | **H** | **N** | **O** |
| **Théorique** | 75,67 | 11,46 | 1,92 | 10,96 |
| **Trouvé** | 75,23 | 11,33 | 1,82 | 11,69 |

### Exemple 3 : Shampooing

- Alkylpolyglucoside vendu sous le nom KAG 40 par la société KAO à 40% de matière active 10 g MA
- Lauryl amido éther carboxylate de sodium (3 OE) vendu sous le nom AKYPO Focum 30 BV par la société CHEM'Y 5 g MA
- Diuréthane d'alcools(C16/C18) oxyéthyléné (6 OE) et oxypropyléné (14 OP) vendu sous le nom ELFACOS T212 2,5 g MA par la société AKZO
- Composé de l'exemple 1 0,1 g
- Conservateur, parfum, HCI pH 6 qs
- Eau q. s. p.100 g

### Exemple 4 : Soin après-shampooing à rincer

- Méthosulfate de 1-méthyl 2-suif 3-suifamido éthylimidazolium/propylène glycol (75/25) vendu par Witco sous le nom de Rewoquat W75PG 2,6 g MA
- Composé de l'exemple 1 0,5 g
- Mélange d'alcool cétylique et cétyl/stéarylique oxyéthyléné 2,5 g
- Conservateur, parfum qs
- Eau q. s. p. 100 g
pH spontané de 3,8

### Exemple 5 : Mesure de la perte insensible en eau (PIE)

Cette mesure s'effectue à l'aide d'un évaporimètre (Servomed) qui détermine quantitativement l'évaporation d'eau, c'est-à-dire un transport d'eau par diffusion, à partir d'un échantillon de stratum corneum préalablement délipidé obturant une capsule cylindrique contenant de l'eau, le tout étant placé dans une chambre à température et humidité relatives contrôlées.
Des capteurs permettent de mesurer la pression partielle de vapeur d'eau en deux points situés à des distances différentes de l'échantillon.
On déterminé ainsi le gradient de pression partielle de vapeur d'eau entre les deux points, et donc le taux d'évaporation conformément à la loi de Fick.
Il a été réalisé un test comparatif des effets sur la P.I.E. d'une solution à 1% en poids en composé A correspondant à celui de l'exemple 1 dans l'isopropanol par rapport à une solution contenant, à la place du composé selon l'invention, le composé B qui est le N(hexadécyloxycarbonyl)-2-amino-octadécane-1,3-diol décrit dans le brevet français 2 652 002

Les résultats sont rassemblés dans le tableau suivant.

| Composé | Composition (concentration) | P.I.E. 20 H (%) |
|---|---|---|
| A (invention) | 1 % dans isopropanol | -6 +/- 2 |
| B (hors invention) | 1 % dans isopropanol | 0 +/- 2 |

On constate donc que l'application des composés selon l'invention permet de réduire de manière significative la perte en eau contenue dans le stratum corneum, démontrant ainsi des propriétés améliorées pour les composés selon l'invention de barrière de perméabilité à l'eau de stratum corneum.

## Revendications

1. Composés répondant à la formule : 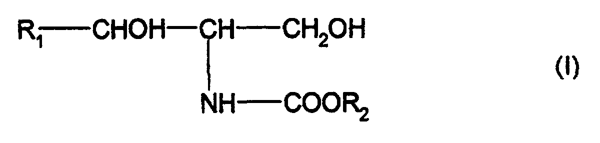 dans laquelle:
* R₁ désigne un radical hydrocarboné en C1 à C31, pouvant être substitué par un ou plusieurs hydroxyles éventuellement estérifiés, à l'exception des composés pour lesquels R1 désigne un radical alkyle linéaire ou ramifié, saturé ou insaturé, éventuellement hydroxylé ayant de 1 à 3 atomes de carbone,
* OR₂ désigne un radical cholestéryle; lesdits composés étant sous forme d'énantiomère pur ou sous forme de mélange d'isomères.

2. Composés selon la revendication 1, caractérisés en ce que R₁ désigne un radical alkyle en C1 à C31, linéaire ou ramifié, saturé ou insaturé pouvant être substitué par un ou plusieurs hydroxyles éventuellement estérifiés.

3. Composés selon la revendication 1 répondant à la formule : 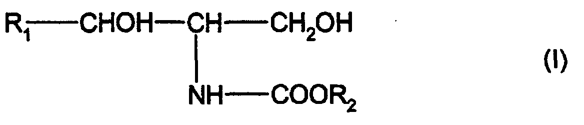 dans laquelle:
* R₁ désigne un radical hydrocarboné en C9 à C25; pouvant être substitué par un ou plusieurs hydroxyles éventuellement estérifiés,
* OR₂ désigne un radical cholestéryle; lesdits composés étant sous forme d'énantiomère pur ou sous forme de mélange d'isomères.

4. Composés selon la revendication 3, caractérisés en ce que R₁ désigne un radical alkyle en C9 à C25, linéaire ou ramifié, saturé ou insaturé pouvant être substitué par un ou plusieurs hydroxyles éventuellement estérifiés.

5. Composés selon l'une quelconque des revendications 1 à 4, caractérisés en ce qu'ils sont choisis parmi :
- le N-(cholestéryloxycarbonyl)-2-amino-octadécane-1,3-diol;
- le N(cholestéryloxycarbonyl)-2-amino-octadécane-1,3,4-triol; lesdits composés étant sous forme pure ou sous forme de mélanges d'isomères.

6. Procédé de préparation des composés de formule (I) définie selon l'une quelconque des revendications 1 à 5, caractérisé en ce qu'il consiste à faire réagir sur la fonction amine d'une base sphingoïde de formule (Il) suivante : 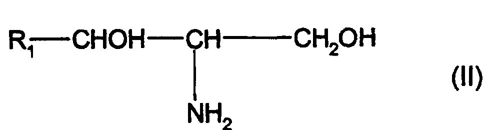 dans laquelle R₁ a la même signification indiquée dans la formule (I)
- soit un chloroformiate de cholestéryle de formule (III) suivante : 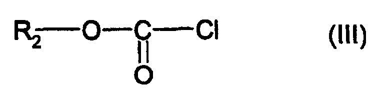 dans laquelle OR₂ a la même signification indiquée dans la formule (I) dans un solvant organique approprié en présence d'une base ;
- soit un imidazolide de cholestéryle de formule (IV) suivante : 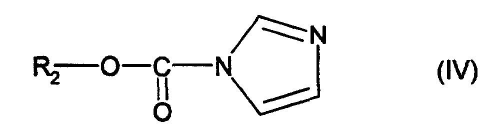 dans laquelle OR₂ a la même signification indiquée dans la formule (I).

7. Composition à usage cosmétique ou dermatologique, caractérisée par le fait qu'elle contient dans un milieu cosmétiquement acceptable au moins un composé lipidique dérivé d'une base sphingoïde tel que défini dans l'une quelconque des revendications 1 à 5.

8. Composition selon la revendication 7, caractérisée par le fait que les composés de formule (I) sont présents dans des quantités allant de 0,005 à 20 % en poids, de préférence de 0,01 à 10 % en poids par rapport au poids total de la composition.

9. Composition selon l'une des revendications 7 à 8, caractérisée par le fait qu'elle se présente sous forme d'émulsion ; de lotion huileuse, oléoalcoolique ou hydroalcoolique ; de gel; de dispersion ; de bâtonnets solides ; de spray ou de mousse aérosol.

10. Composition à usage cosmétique ou dermatologique, caractérisée par le fait qu'elle se présente sous forme d'une dispersion aqueuse de sphérules lipidiques, constituées de couches moléculaires organisées renfermant une phase aqueuse encapsulée, ces couches étant constituées d'au moins un composé de formule (I) tel que défini dans l'une quelconque des revendications 1 à 5 associé à au moins un autre composé lipidique.

11. Composition sous forme de dispersion aqueuse de sphérules lipidiques selon la revendication 10, caractérisée par le fait que l'autre composé lipidique est choisi parmi les alcools et diols à longue chaîne, les stérols, les phospholipides, les glycolipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quatemaire, les esters phosphoriques d'alcools gras, les alkylsulfates et les acides gras sous forme de sels.

12. Composition sous forme de dispersion aqueuse de sphérules lipidiques selon la revendication 10 ou 11, caractérisée par le fait que les sphérules ont un diamètre compris entre 0,05 et 5 microns.

13. Utilisation d'un composé de formule (I) selon l'une quelconque des revendications 1 à 5, comme agent hydratant dans une formulation à usage cosmétique ou pour la fabrication de formulations à usage dermatologique.

14. Utilisation d'un composé de formule (I) selon l'une quelconque des revendications 1 à 5 en association avec au moins un autre composé lipidique, pour la formation de dispersions de sphérules lipidiques à usage cosmétique ou dermatologique.

15. Procédé de traitement cosmétique hydratant de la peau, des cheveux, des ongles ou des cils, caractérisé par le fait qu'il consiste à appliquer sur la peau, les cheveux, les ongles ou les cils, une composition selon l'une quelconque des revendications 7 à 12.

## Patentansprüche

1. Verbindungen der Formel: 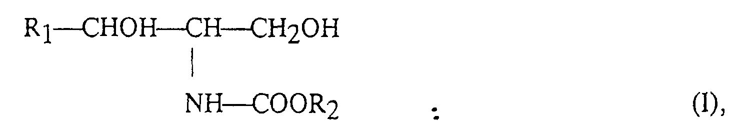 worin bedeuten:
- R₁ eine Kohlenwasserstoffgruppe mit 1 bis 31 Kohlenstoffatomen, die mit einer oder mehreren gegebenenfalls veresterten Hydroxygruppen substituiert sein kann, wobei Verbindungen ausgenommen sind, worin R₁ eine geradkettige oder verzweigte, gesättigte oder ungesättigte und gegebenenfalls hydroxylierte Alkylgruppe bedeutet, die 1 bis 3 Kohlenstoffatome aufweist,
und
- OR₂ eine Cholesterylgruppe,
wobei diese Verbindungen in Form eines reinen Enantiomers oder in Form von Isomerengemischen vorliegen.

2. Verbindungen nach Anspruch 1, dadurch gekennzeichnet, dass R₁ eine geradkettige oder verzweigte, gesättigte oder ungesättigte C₁₋₃₁-Alkylgruppe bedeutet, die mit einer oder mehreren gegebenenfalls veresterten Hydroxygruppen substituiert sein kann.

3. Verbindungen nach Anspruch 1 der Formel: 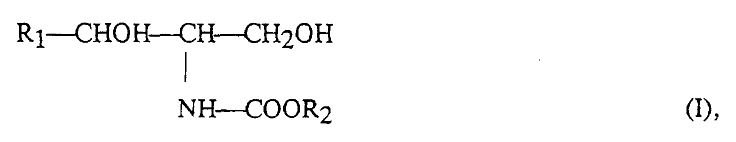 worin bedeuten:
- R₁ eine Kohlenwasserstoffgruppe mit 9 bis 25 Kohlenstoffatomen, die mit einer oder mehreren gegebenenfalls veresterten Hydroxygruppen substituiert sein kann,
und
- OR₂ eine Cholesterylgruppe,
wobei diese Verbindungen in Form eines reinen Enantiomers oder in Form von Isomerengemischen vorliegen.

4. Verbindungen nach Anspruch 3, dadurch gekennzeichnet, dass R₁ eine geradkettige oder verzweigte, gesättigte oder ungesättigte C₉₋₂₅-Alkylgruppe bedeutet, die mit einer oder mehreren gegebenenfalls veresterten Hydroxygruppen substituiert sein kann.

5. Verbindungen nach einem der Ansprüche 1 bis 4, dadurch gekennzeichnet, dass sie ausgewählt sind unter:
- N-(Cholesteryloxycarbonyl)-2-amino-octadecan-1,3-diol und
- N-(Cholesteryloxycarbonyl)-2-amino-octadecan-1,3,4-triol,
wobei die Verbindungen in reiner Form oder in Form von Isomerengemischen vorliegen.

6. Verfahren zur Herstellung von Verbindungen der Formel (I) nach einem der Ansprüche 1 bis 5, dadurch gekennzeichnet, dass es darin besteht, dass mit der Aminogruppe eines Sphingoids der folgenden Formel (II): 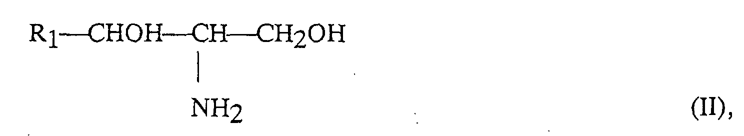 worin R₁ die in Formel (I) angegebene Bedeutung aufweist,
- entweder in Gegenwart einer Base in einem geeigneten organischen Lösemittel ein Cholesterylchlorformiat der folgenden Formel (III): 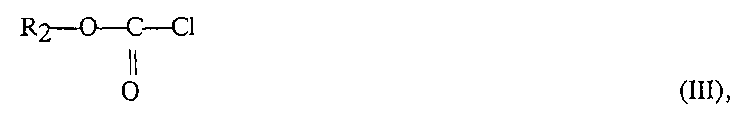 worin OR₂ die in Formel (I) angegebene Bedeutung aufweist,
- oder ein Cholesterylimidazolid der folgenden Formel (IV) umgesetzt wird: 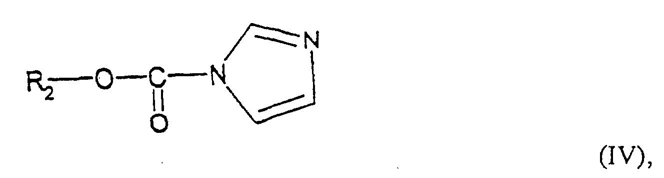 worin OR₂ die in Formel (I) angegebene Bedeutung aufweist.

7. Zusammensetzung zur kosmetischen oder dermatologischen Anwendung, dadurch gekennzeichnet, dass sie in einem kosmetisch akzeptablen Medium mindestens eine wie in einem der Ansprüche 1 bis 5 definierte Lipidverbindung enthält, die von einem Sphingoid abgeleitet ist.

8. Zusammensetzung nach Anspruch 7, dadurch gekennzeichnet, dass die Verbindungen der Formel (I) in Mengenanteilen im Bereich von 0,005 bis 20 Gew.-% und vorzugsweise von 0,01 bis 10 Gew.-%, bezogen auf das Gesamtgewicht der Zusammensetzung, vorliegen.

9. Zusammensetzung nach einem der Ansprüche 7 bis 8, dadurch gekennzeichnet, dass sie in Form einer Emulsion, einer öligen, ölig-alkoholischen oder wässerig-alkoholischen Lotion, eines Gels, einer Dispersion, fester Stifte, eines Sprays oder eines Aerosolschaums vorliegt.

10. Zusammensetzung zur kosmetischen oder dermatologischen Anwendung, dadurch gekennzeichnet, dass sie in Form einer wässerigen Dispersion von Lipidkügelchen vorliegt, die aus organisierten molekularen Schichten bestehen, die eine eingekapselte wässerige Phase umschließen, wobei die Schichten aus mindestens einer Verbindung der Formel (I) nach einem der Ansprüche 1 bis 5 in Kombination mit mindestens einer weiteren Lipidverbindung bestehen.

11. Zusammensetzung in Form einer wässerigen Dispersion von Lipidkügelchen nach Anspruch 10, dadurch gekennzeichnet, dass die weitere Lipidverbindung ausgewählt ist unter langkettigen Alkoholen und Diolen, Sterinen, Phospholipiden, Glykolipiden, Cholesterylsulfat, Cholesterylphosphat, langkettigen Aminen und ihren quartären Ammoniumderivaten, Dihydroxyalkylaminen, polyethoxylierten Fettaminen, Estern von Aminoalkoholen mit langer Kette, ihren Salzen und quartären Ammoniumderivaten, Phosphorsäureestern von Fettalkoholen, Alkylsulfaten und Fettsäuren in Form von Salzen.

12. Zusammensetzung in Form einer wässerigen Dispersion von Lipidkügelchen nach Anspruch 10 oder 11, dadurch gekennzeichnet, dass die Kügelchen einen Durchmesser im Bereich von 0,05 bis 5 Mikrometern aufweisen.

13. Verwendung einer Verbindung der Formel (I) nach einem der Ansprüche 1 bis 5 als Feuchthaltemittel in einer Formulierung zur kosmetischen Anwendung oder zur Herstellung von Formulierungen zur dermatologischen Anwendung.

14. Verwendung einer Verbindung der Formel (I) nach einem der Ansprüche 1 bis 5 in Kombination mit mindestens einer weiteren Lipidverbindung zur Bildung von Dispersionen von Lipidkügelchen zur kosmetischen oder dermatologischen Anwendung.

15. Verfahren zur hydratisierenden kosmetischen Behandlung der Haut, der Haare, der Nägel oder der Wimpern, dadurch gekennzeichnet, dass es darin besteht, eine Zusammensetzung nach einem der Ansprüche 7 bis 12 auf die Haut, die Haare, die Nägel oder die Wimpern aufzutragen.

## Claims

1. Compounds corresponding to the formula: 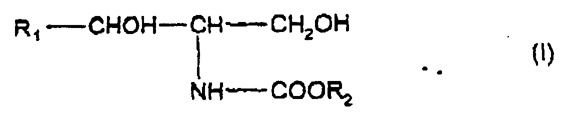 in which:
* R₁ denotes a C₁ to C₃₁ hydrocarbonaceous radical which can be substituted by one or more optionally esterified hydroxyls, with the exception of the compounds for which R₁ denotes an optionally hydroxylated, saturated or unsaturated, linear or branched alkyl radical having from 1 to 3 carbon atoms,
* OR₂ denotes a cholesteryl radical; the said compounds being in the form of a pure enantiomer or in the form of a mixture of isomers.

2. Compounds according to Claim 1, characterized in that R₁ denotes a saturated or unsaturated, linear or branched, C₁ to C₃₁ alkyl radical which can be substituted by one or more optionally esterified hydroxyls.

3. Compounds according to Claim 1, corresponding to the formula: 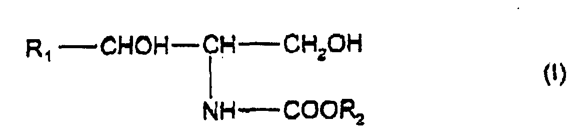 in which:
* R₁ denotes a C₉ to C₂₅ hydrocarbonaceous radical which can be substituted by one or more optionally esterified hydroxyls,
* OR₂ denotes a cholesteryl radical; the said compounds being in the form of a pure enantiomer or in the form of a mixture of isomers.

4. Compounds according to Claim 3, characterized in that R₁ denotes a saturated or unsaturated, linear or branched, C₉ to C₂₅ alkyl radical which can be substituted by one or more optionally esterified hydroxyls.

5. Compounds according to any one of Claims 1 to 4, characterized in that they are chosen from:
- N-(cholesteryloxycarbonyl)-2-aminooctadecane-1,3-diol ;
- N-(cholesteryloxycarbonyl)-2-aminooctadecane-1,3,4-triol;
the said compounds being in the pure form or in the form of mixtures of isomers.

6. Process for the preparation of the compounds of formula (I) defined according to any one of Claims 1 to 5, characterized in that it consists in reacting the amine functional group of a sphingoid base of following formula (II) : 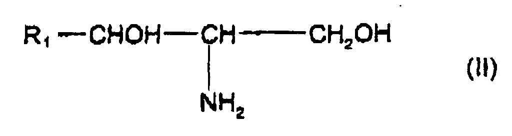 in which R₁ has the same meaning indicated in the formula (I)
- either with a cholesteryl chloroformate of following formula (III) : 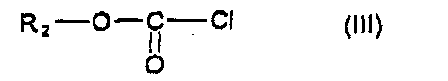 in which OR₂ has the same meaning indicated in the formula (I), in an appropriate organic solvent in the presence of a base;
- or with a cholesteryl imidazolide of following formula (IV) : 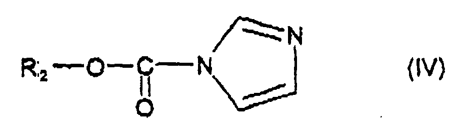 in which OR₂ has the same meaning indicated in the formula (I).

7. Composition for cosmetic or dermatological use, characterized in that it comprises, in a cosmetically acceptable medium, at least one lipid compound derived from a sphingoid base as defined in any one of Claims 1 to 5.

8. Composition according to Claim 7, characterized in that the compounds of formula (I) are present in amounts ranging from 0.005 to 20% by weight, preferably from 0.01 to 10% by weight, with respect to the total weight of the composition.

9. Composition according to either of Claims 7 and 8, characterized in that it is provided in the form of an emulsion; of an aqueous/alcoholic, oleoalcoholic or oily lotion; of a gel; of a dispersion; of solid sticks; or of an aerosol foam or spray.

10. Composition for cosmetic or dermatological use, characterized in that it is provided in the form of an aqueous dispersion of lipid spherules composed of organized molecular layers enclosing an encapsulated aqueous phase, these layers being composed of at least one compound of formula (I) as defined in any one of Claims 1 to 5 in combination with at least one other lipid compound.

11. Composition in the form of an aqueous dispersion of lipid spherules according to Claim 10, characterized in that the other lipid compound is chosen from long-chain alcohols and diols, sterols, phospholipids, glycolipids, cholesteryl sulphate, cholesteryl phosphate, long-chain amines and their quaternary ammonium derivatives, dihydroxyalkylamines, polyoxyethylenated fatty amines, long-chain aminoalcohol esters, their salts and quaternary ammonium derivatives, phosphoric esters of fatty alcohols, alkyl sulphates and fatty acids in the form of salts.

12. Composition in the form of an aqueous dispersion of lipid spherules according to Claim 10 or 11, characterized in that the spherules have a diameter of between 0.05 and 5 microns.

13. Use of a compound of formula (I) according to any one of Claims 1 to 5 as moisturizing agent in a formulation for cosmetic use or for the manufacture of formulations for dermatological use.

14. Use of a compound of formula (I) according to any one of Claims 1 to 5, in combination with at least one other lipid compound, for the formation of dispersions of lipid spherules for cosmetic or dermatological use.

15. Process for the cosmetic moisturizing treatment of the skin, hair, nails or eyelashes, characterized in that it consists in applying, to the skin, hair, nails or eyelashes, a composition according to any one of Claims 7 to 12.
